# FASCICULE DE BREVET EUROPEEN

(11) **EP 2 904 964 B1**
(45) Date de publication et mention de la délivrance du brevet: **02.10.2019**
(21) Numéro de dépôt: 15152441.0
(22) Date de dépôt: 26.01.2015
(51) Int. Cl.: A61B 5/0205, A61B 5/0402, A61B 5/00, A61B 5/0452, G16H 40/63, G16H 50/30, G16H 50/20, G16H 20/30, A61B 5/1455, A61B 7/04, A61B 5/08, A61B 5/024

(54) **Ensemble avec dispositif médical à stimulation kinesthésique pour l'évaluation non invasive de l'équilibre sympathovagal d'un patient.**
Kit aus einer medizinischen Vorrichtung mit kinästhetischer Stimulation für die nicht-invasive Beurteilung des sympathovagalen Gleichgewichts eines Patienten
Assembly including a medical device with kinaesthetic stimulation for non-invasive assessment of the sympathovagal balance of a patient

(30) Priorité: 11.02.2014 FR 1400374
(43) Date de publication de la demande: 12.08.2015
(73) Titulaire: Sorin CRM SAS, 92143 Clamart Cedex (FR); Université de Rennes 1, 35065 Rennes Cedex (FR)
(72) Inventeur: Bonnet, Jean-Luc, 91300 Massy (FR); Hernandez, Alfredo, 35510 Cesson Sévigné (FR)
(74) Mandataire: Grünecker Patent- und Rechtsanwälte PartG mbB

(56) Documents cités:
- US-A1- 2007 239 210
- US-A1- 2008 281 372
- US-A1- 2010 268 104

## Description

L'invention concerne l'évaluation de l'équilibre sympathovagal chez un patient.

L'équilibre sympathovagal ou SVB (*Sympatho-Vagal Balance*) est l'équilibre entre les deux branches sympathique et parasympathique du système nerveux autonome. Cet équilibre peut jouer un rôle important dans un certain nombre de pathologies telles que l'insuffisance cardiaque, l'infarctus du myocarde ou les syndromes cardio-respiratoires, de sorte que son évaluation peut constituer un outil important pour le diagnostic, le suivi et le choix d'une thérapie pour les pathologies de ce type.

On sait en effet que le système cardiovasculaire est sous le contrôle à la fois du système sympathique et du système parasympathique. Chez un patient normal, ces deux systèmes sont équilibrés, ce qui se traduit notamment par un rythme cardiaque bien adapté à l'activité courante du patient (effet chronotrope), une contractilité cardiaque satisfaisante (effet inotrope), etc.

Chez un patient souffrant d'insuffisance cardiaque, ou dans le post-infarctus du myocarde, l'activité du système sympathique est excessive (état sympathique hypertonique), avec au contraire un système parasympathique déprimé, ce qui conduit à un rythme cardiaque plus rapide que la normale. L'équilibre sympathovagal est toutefois difficile à déterminer, en particulier dans des situations de crise aigüe ou en présence d'une pathologie d'évolution chronique, et/ou si l'on veut évaluer cet équilibre à intervalles réguliers, par exemple quotidiennement au moyen d'un dispositif externe ou implanté assurant en continu le *monitoring* du patient.

La manière conventionnelle d'évaluer la situation du système nerveux autonome d'un patient, et donc son équilibre sympathovagal, est souvent basée sur l'analyse d'indicateurs de la variabilité du rythme cardiaque HRV (*Heart Rate Variability*) dans le domaine fréquentiel ou temporel.

L'évaluation de l'équilibre sympathovagal prévoit le plus souvent l'application de "manoeuvres autonomiques", c'est-à-dire d'actions permettant de déclencher une réponse du système nerveux autonome du patient ("réponse autonomique").

Les manoeuvres autonomiques les plus courantes mettent en oeuvre des moyens pharmacologiques ou des manipulations mécaniques (manoeuvre de Valsalva, test d'inclinaison ou *tilt test*) permettant de créer une modification contrôlée du système cardiovasculaire destinée à provoquer une réponse du système nerveux autonome, qui est recueillie et analysée. Une évaluation directe de l'activité du système nerveux autonome est également possible de façon directe au niveau cérébral, mais par des techniques fortement invasives.

Ces différentes manoeuvres ne peuvent pas être appliquées dans le contexte d'un suivi régulier d'un patient au moyen d'un système implanté ou ambulatoire, car ceci impliquerait de demander au patient d'exécuter ces manoeuvres autonomes de façon répétée et reproductible, ce qui en outre est souvent impossible en raison de l'état pathologique du patient, ou à plus forte raison pendant son sommeil (qui est la période la plus favorable à l'évaluation de l'équilibre sympathovagal compte tenu de la moindre incidence des facteurs extérieurs).

Divers procédés, tels que celui divulgué par le US 2008/0281372 A1, ont été proposés pour analyser la variabilité HRV suite à des perturbations spontanées ou induites du système cardiovasculaire, sans participation active du patient. En particulier, l'analyse de la turbulence du rythme cardiaque HRT (*Heart Rate Turbulence*) utilise comme perturbation la survenue d'un battement ectopique spontané. Bien que ce procédé puisse être aisément appliqué à un système implantable ou externe pour un suivi régulier de l'équilibre sympathovagal, il repose sur des événements de nature imprévisible (les battements ectopiques), qui peuvent survenir à des moments aléatoires, ou pas du tout, provoquant un biais important sur l'estimation de l'équilibre sympathovagal. Il a également été proposé d'appliquer une stimulation ventriculaire pour reproduire l'évènement ectopique servant de perturbation cardiovasculaire pour l'analyse de la réponse autonome. Cette solution, basée sur la production d'un battement ectopique, est cependant délétère pour notamment des patients en insuffisance cardiaque.

En tout état de cause, ces techniques présupposent l'existence d'un dispositif implanté chez le patient (stimulateur cardiaque ou analogue), ce qui réduit fortement la population de patients auxquels elles peuvent être appliquées.

Le besoin subsiste donc de disposer d'un appareil externe, non invasif, permettant d'évaluer l'équilibre SVB d'un patient quelconque, par exemple au moyen d'un enregistreur de type Holter ne nécessitant aucune autre intervention que la pose d'électrodes, capteurs ou autres transducteurs sur le corps du patient et leur raccordement à un appareil porté en ambulatoire par ce dernier.

À cet égard, il a été proposé des techniques qui ne sont pas basées sur le rythme cardiaque du patient. Il s'agit notamment d'analyses de la variabilité de la pression sanguine ou encore de la contractilité cardiaque, lorsque ces informations sont disponibles. Mais dans ces propositions, il est toujours nécessaire de provoquer des manoeuvres autonomes reproductibles pour obtenir des estimations fiables de l'évolution de l'équilibre sympathovagal.

Le besoin subsiste ainsi d'un ensemble permettant d'évaluer de façon entièrement automatique et non invasive l'équilibre sympathovagal, sans participation du patient ni d'un soignant.

Il serait à cet égard particulièrement avantageux de pouvoir disposer d'un dispositif qui soit compatible avec un suivi ou un monitoring clinique de longue durée, en délivrant par exemple quotidiennement un indice ou indicateur représentatif de l'équilibre sympathovagal du patient à partir de signaux cardiovasculaires recueillis par le dispositif en réponse à une modification contrôlée, reproductible et non délétère, du système nerveux autonome du patient.

Le US 2010/0268104 A1 divulgue un tel dispositif, qui stimule le système auditif du patient par un transducteur acoustique émettant dans l'oreille de ce dernier des tonalités plus ou moins hautes et plus ou moins fortes, et qui simultanément recueille un électrocardiogramme qui est analysé pour évaluer la variabilité HRV.

L'invention propose un dispositif d'une autre nature, reposant sur l'utilisation d'un effecteur kinesthésique, c'est-à-dire d'un vibreur posé contre l'épiderme du patient et produisant sur la peau une stimulation mécanique vibratile, qui est détectée par les récepteurs sensoriels ou mécanorécepteurs de l'organisme et transmise au système nerveux central autonome par l'intermédiaire des nerfs sensitifs.

Plus précisément, l'invention propose à cet effet un ensemble selon la revendication 1.

Dans une forme de réalisation avantageuse :
- pendant la période de test, le générateur produit une pluralité N de salves d'impulsions de stimulation kinesthésique ;
- pendant une période incluant ladite période de récupération, les moyens de mesure sont aptes à mesurer la valeur courante du au moins un paramètre témoin en synchronisme avec le rythme cardiaque du patient pendant une pluralité de cycles cardiaques successifs, donnant pour chaque salve *n* échantillons distincts du au moins un paramètre témoin de rang *i* respectif, avec *i* = 1 ... *n* ; et
- les moyens évaluateurs sont aptes à calculer une moyenne des *N* échantillons de même rang *i* du au moins un paramètre témoin pour une même période de test, donnant ainsi *n* valeurs moyennées du paramètre témoin, l'ensemble de ces *n* valeurs moyennées étant l'indice SVB de la période de test.

L'ensemble peut par ailleurs comprendre des moyens aptes à inhiber les moyens d'activation contrôlée du générateur en cas de détection par le dispositif de la survenue d'au moins un événement parmi : fréquence cardiaque inférieure à un seuil prédéterminé, présence d'une activité physique du patient, délivrance d'une stimulation cardiaque, présence d'un épisode d'apnée ou d'hypopnée, présence d'un épisode d'arythmie.

Le paramètre témoin de l'activité autonomique courante du patient peut être notamment au moins l'un d'entre : l'intervalle RR comme paramètre chronotropique ; l'intervalle PR comme paramètre dromotropique ; et un paramètre d'accélération endocardiaque EA comme paramètre inotropique.

On va maintenant décrire un exemple de mise en oeuvre de la présente invention, en référence aux dessins annexés où les mêmes références désignent d'une figure à l'autre des éléments identiques ou fonctionnellement semblables.
La Figure 1 est une représentation schématique des différents éléments de l'ensemble d'évaluation de l'équilibre sympathovagal selon l'invention.
La Figure 2 est une représentation schématique du principe sur lequel est basé l'ensemble selon l'invention pour l'évaluation de l'équilibre sympathovagal.
La Figure 3 est un exemple de signaux d'activité cardiaque enregistrés chez un patient lors de la délivrance de salves de stimulation kinesthésique (stimulation vibrotactile).
La Figure 4 illustre un exemple de salves de stimulation kinesthésique utilisées pour la mise en oeuvre de l'invention.

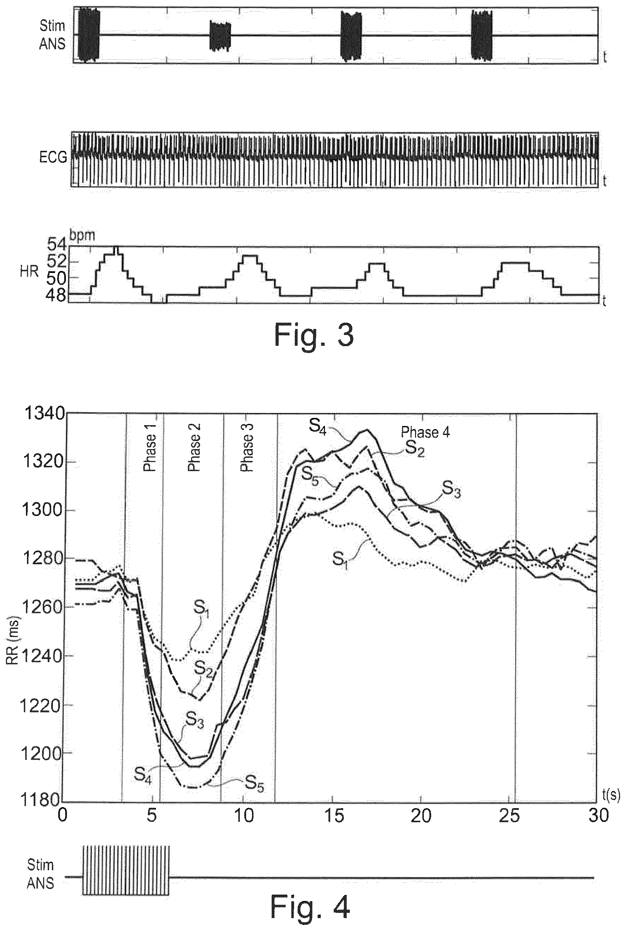

La Figure 4 illustre la réponse moyenne des cycles RR consécutifs à des stimulations kinesthésiques de différentes amplitudes sur un sujet sain, avec les différentes phases de cette réponse.

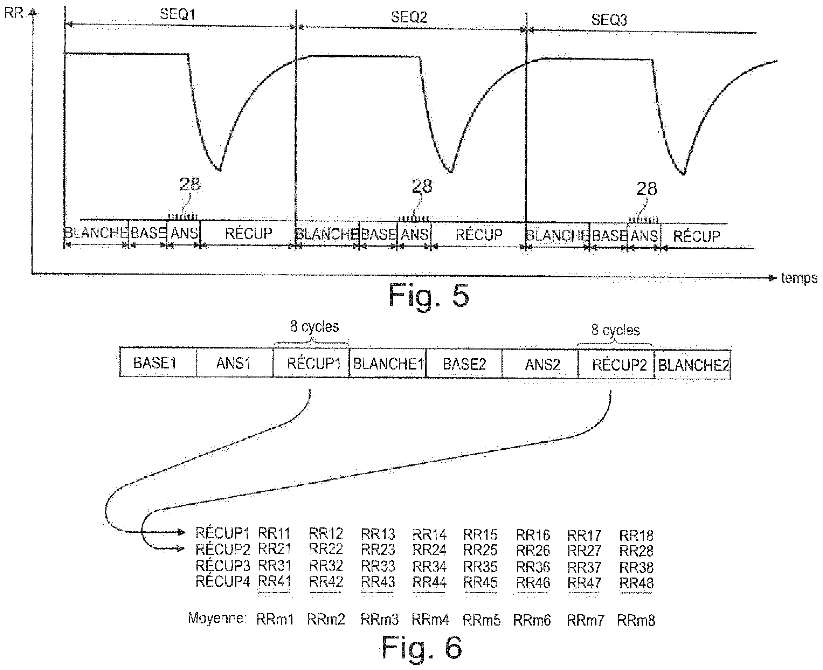

La Figure 5 illustre les variations des distances RR en réponse à l'application de salves telles que celles de la Figure 4, au cours de trois périodes de test successives.
La Figure 6 illustre la manière dont les données de rythme cardiaque sont recueillies, mémorisées et moyennées pour permettre d'évaluer l'état physiologique du patient.

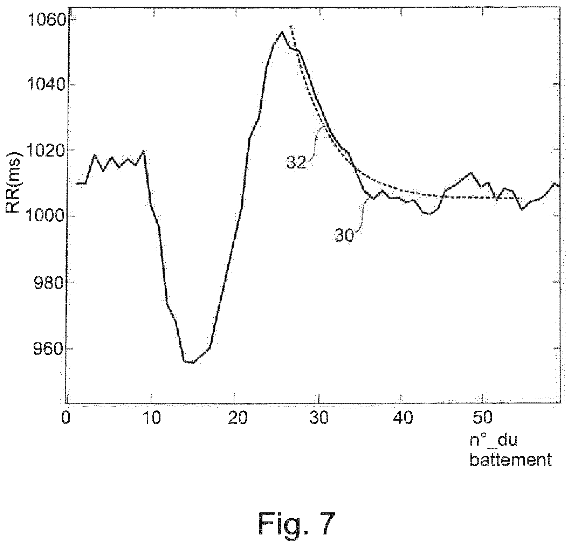

La Figure 7 est un exemple d'analyse par recherche d'une approximation exponentielle appliquée à des points dérivés des relevés de mesure du rythme cardiaque lors de l'application de la modification de l'équilibre autonomique.

On va maintenant décrire un exemple de réalisation de l'invention.

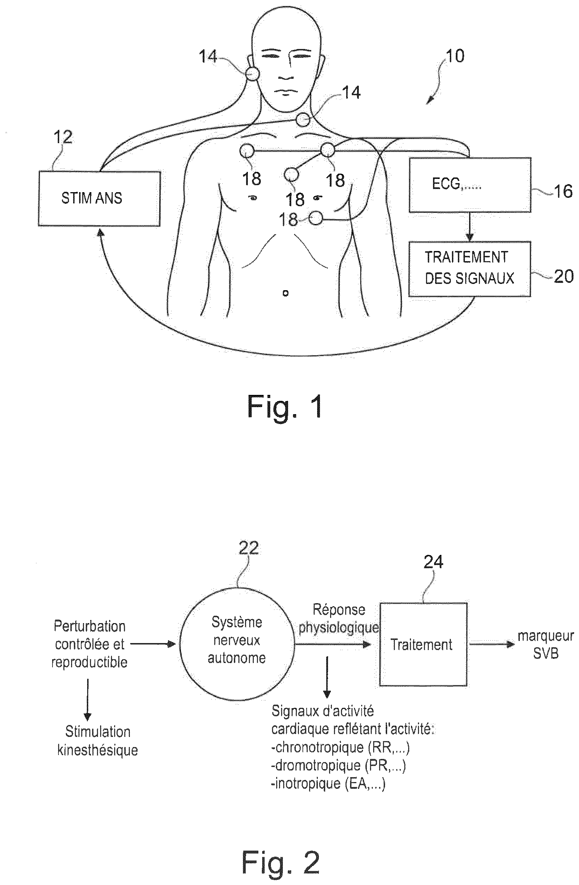

Sur la Figure 1, la référence 10 désigne de façon générale l'ensemble selon l'invention permettant d'évaluer l'équilibre sympathovagal SVB d'un patient.

Le système comporte un dispositif de stimulation avec un boitier générateur 12 produisant des impulsions appliquées à un (ou plusieurs) effecteur(s) 14, constitué par exemple d'un vibreur placé dans une région sensible de l'épiderme, typiquement (chez l'adulte) dans la région de l'os mastoïde au voisinage de l'oreille. La stimulation vibrotactile appliquée sur la peau par l'effecteur 14, détectée par les récepteurs sensoriels ou mécanorécepteurs de l'organisme, sera transmise au système nerveux central autonome par l'intermédiaire des nerfs sensitifs. Ce type de stimulation vibrotactile opérée par voie cutanée va engendrer une perturbation contrôlée du système autonomique, et sera ici désignée "stimulation kinesthésique" ou encore "stimulation ANS" (*Autonomic Nervous Stimulation*).

L'effecteur 14 est par exemple un transducteur du type *C10-100* de Precision Microdrives ou *C2 Tactor* de Engineering Acoustics. Il s'agit d'un transducteur de quelques grammes susceptible d'émettre des vibrations grâce à un vibreur intégré excité par des trains d'impulsions d'amplitude et de durée variables, typiquement à une fréquence de 250 Hz qui est la fréquence nominale de résonance de cet effecteur particulier et qui est également la fréquence à laquelle les mécanorécepteurs de la peau sont les plus sensibles. D'autres types d'effecteur peuvent bien entendu être efficacement utilisés.

Le générateur 12 est piloté par un microcontrôleur et possède des moyens permettant de régler l'intensité (c'est-à-dire l'énergie) de la stimulation kinesthésique, par variation contrôlée de l'amplitude et/ou du nombre, de la durée et/ou de la fréquence de stimulation des trains d'impulsions formant le signal appliqué à l'effecteur 14.

Le système 10 comporte en outre un enregistreur de type Holter avec un module 16 d'acquisition de signaux relié à divers capteurs ou électrodes 18 permettant de mesurer des signaux physiologiques tels que l'ECG, la fréquence cardiaque, la respiration, la saturation en oxygène, l'onde de pouls, le phonocardiogramme, etc.

Dans la suite, on s'intéressera principalement aux signaux physiologiques directement liés à l'activité cardiaque, notamment la fréquence cardiaque qui est un paramètre simple à obtenir à partir d'un signal ECG. Mais ce choix n'est pas limitatif, et l'invention peut être mise en oeuvre à partir d'autres signaux physiologiques recueillis de manière non invasive sur le corps du patient.

En particulier, l'accélération endocardiaque (EA) du myocarde est un signal susceptible de fournir divers paramètres significatifs reflétant l'activité du coeur. Ce signal EA peut être obtenu par voie externe au moyen d'un accéléromètre captant un signal phonocardiographique. On pourra par exemple se référer au EP 1 741 387 A1 (ELA Medical) qui décrit un appareillage de recueil et de traitement de tels signaux chez un patient, de manière non invasive, avec en particulier la manière d'en extraire divers paramètres représentatifs tels que l'amplitude de crête du pic d'accélération endocardiaque (PEA), l'intervalle temporel séparant les deux pics PEA1 et PEA2 correspondant aux deux bruits principaux du coeur, etc. D'autres signaux encore peuvent être recueillis, notamment par acquisition de biopotentiels de surface tels que ECG, ENG, EEG et EMG (potentiels respectivement cardiaque, nerveux, encéphalographique et musculaire).

Le module d'acquisition 16 inclut tous les composants nécessaires à l'amplification et au filtrage des signaux physiologiques recueillis, qui sont ensuite appliqués à un module 20 de traitement des signaux destiné à extraire, comme on l'exposera par la suite, un ensemble de paramètres spécifiques porteurs d'une information représentative de l'équilibre SVB, en particulier procurant une information sur l'effet produit sur le système cardiovasculaire par la perturbation autonomique résultant de la stimulation kinesthésique.

Le module 20 est par ailleurs couplé au générateur kinesthésique 12 de manière à ajuster un certain nombre de paramètres de fonctionnement de celui-ci, tout particulièrement le moment de délivrance d'une salve d'impulsions. Les signaux ECG recueillis par le module 16 permettent ainsi de piloter le générateur 12 afin que celui-ci puisse délivrer à l'effecteur kinesthésique 14 des salves d'impulsions ANS au moment le plus approprié de l'onde de dépolarisation cardiaque.

L'appareil Holter peut également comprendre des moyens de couplage, filaire ou sans fil, vers un dispositif externe, par exemple un programmateur permettant de recueillir des données d'un enregistrement, ou encore un système de *home monitoring* permettant de recueillir à intervalles réguliers les données relatives au patient et transmettre ces données à un centre distant pour analyse en différé.

La Figure 2 illustre le principe de l'invention qui, pour évaluer l'équilibre sympathovagal du patient, prévoit de générer des modifications contrôlées et reproductibles du système nerveux autonome par des stimulations à prédominance sympathique telles que des stimulations kinesthésiques (ANS).

Ces modifications appliquées au système nerveux autonome 22 produisent une réponse physiologique, mesurable sur un certain nombre de signaux. Les signaux recueillis font l'objet d'un traitement 24 permettant d'obtenir un indice ou marqueur fiable de l'état de l'équilibre sympathovagal du patient et de l'évolution de cet équilibre dans le temps.

Plus précisément, pour appliquer la modification contrôlée du système nerveux autonome à un instant donné, prédéfini ou calculé par le dispositif, un algorithme de stimulation est initié, qui produit une séquence d'impulsions ANS, décrite plus en détail en référence aux Figures 3 et 4 ci-après.

Une stimulation ANS sympathique (comme celle produite par une stimulation kinesthésique) produit sur l'activité cardiaque un certain nombre d'effets, qui peuvent être de nature :
- chronotrope : augmentation du rythme cardiaque, c'est-à-dire diminution des intervalles RR ;
- dromotrope : augmentation de la vitesse de conduction AV, conduisant à une diminution des intervalles PR ;
- bathmotrope : augmentation de l'excitabilité des myosites ;
- inotrope : augmentation de la contractilité cardiaque ; et/ou
- lusitrope : diminution de la vitesse de relaxation cardiaque.

La stimulation ANS a également un effet sur le système vasculaire par modulation de la vasoconstriction, avec une modification des diamètres des artères et de la résistance périphérique se traduisant par une vasoconstriction générale du système vasculaire. Les effets inverses sont généralement produits par une stimulation parasympathique (vagale).

La Figure 3 illustre un exemple de signaux d'activité cardiaque (électrocardiogramme ECG et rythme cardiaque HR) enregistrés chez un patient appareillé avec un équipement tel que celui de la Figure 1, lors de la délivrance de salves de stimulation ANS (chronogramme référencé "Stim ANS").

La stimulation ANS est dans cet exemple constituée d'une séquence de quatre salves d'amplitudes différentes, avec pour chacune une stimulation d'une durée de 5 s à une fréquence fixe de 250 Hz et des périodes "blanches" de 30 s séparant chacune de ces salves. Dans cet exemple, la stimulation est donc réalisée de façon asynchrone par rapport au rythme cardiaque. L'effet chronotrope positif généré par la stimulation vibrotactile (à prédominance sympathique) est clairement visible sur le signal HR, chaque stimulation provoquant à chaque fois une augmentation de la fréquence cardiaque de plusieurs battements/minute (bpm).

De façon générale, si l'on considère le rythme cardiaque, par exemple reflété par la variation de l'intervalle RR, la réponse chronotrope à une stimulation ANS sympathique se traduit typiquement par quatre phases successives.

Ces phases successives sont notamment visibles sur la Figure 4, qui illustre la réponse moyenne des cycles RR consécutifs à des stimulations kinesthésiques de différentes amplitudes sur un sujet sain.

L'allure typique de la réponse chronotrope à ce type de stimulation comprend quatre phases distinctes :
- phase n° 1 : réduction de l'intervalle RR (augmentation du signal HR) ;
- phase n° 2 : phase de pseudo-plateau, avec augmentation plus ou moins visible des intervalles RR ;
- phase n° 3 : augmentation rapide de l'intervalle RR ;
- phase n° 4 : phase de "rebond", qui reflète les réponses transitoires baroréflexes, impliquant à la fois les systèmes sympathique et parasympathique et conduisant à une stabilisation finale de la pression sanguine et du rythme cardiaque.

Cette figure montre également un effet "dose-réponse" : plus l'amplitude de stimulation est importante, plus l'amplitude pic-à-pic et donc les pentes de la réponse sont élevées (les réponses S₁ ... S₅ correspondant à des stimulations d'amplitudes respectives croissantes). On notera par ailleurs que le déphasage entre le début de la stimulation ANS et le début de la réponse chronotrope est variable entre les individus.

Les quatre phases 1 à 4 ci-dessus peuvent également être observées sur la réponse inotropique (variation de contractilité cardiaque) ainsi que sur les autres types de réponse, mais avec une dynamique plus lente due à des constantes de temps plus importantes. Pour cette raison on choisira de préférence - mais de façon non limitative - d'analyser le rythme cardiaque (intervalles RR) pour évaluer selon l'invention l'équilibre sympatho-vagal du patient.

La stimulation ANS peut être définie par de nombreux paramètres de configuration tels que :
- synchronisme : la stimulation ANS peut être synchrone des évènements cardiaques (comme dans le présent exemple), ou désynchronisée de ceux-ci ;
- dans le cas d'une stimulation synchrone, ratio entre les stimulations ANS et les événements cardiaques (un ratio de 1:1 indiquant une stimulation ANS à chaque événement cardiaque détecté, un ratio de 1:4 indiquant une impulsion de stimulation ANS tous les quatre évènements cardiaques, etc.) ;
- dans le cas d'une stimulation ANS synchrone, intervalle R-ANS : ce paramètre décrit le retard entre la détection de l'évènement cardiaque et le début de la salve d'impulsions ANS ;
- tension efficace (RMS) (ou amplitude) délivrée à l'effecteur kinesthésique ;
- fréquence de stimulation ANS de chaque salve ;
- fréquence intersalve ;
- largeur des impulsions ;
- nombre de répétitions de la séquence d'impulsions ;
- rapport cyclique (*duty cycle*) représentant l'alternance de périodes de stimulation et de non-stimulation.

Dans l'exemple particulier illustré Figure 3, la stimulation ANS comprend des stimulations asynchrones avec une fréquence de stimulation de 250 Hz (proche de la fréquence de réponse optimale des mécanorécepteurs), d'une durée de 5 s, une durée intersalve de 30 s et d'amplitude variable.

La Figure 5 illustre la succession des séquences d'analyse des variations du rythme cardiaque (évalué par la mesure de l'intervalle RR) en réponse aux salves ANS successives 28.

Chacune de ces séquences (SEQ1, SEQ2 ...) comprend :
- une période blanche (BLANCHE) permettant au système nerveux autonome de retourner à son état de base après la modification résultant de la stimulation ANS à la séquence précédente. Pendant cette période, la stimulation ANS est désactivée ;
- une période de "ligne de base" (BASE), servant à l'enregistrement d'un état stationnaire des signaux d'activité cardiaque juste avant l'application de la stimulation ANS.
   Pendant cette période, le système peut vérifier par ailleurs si un certain nombre de critères sont remplis, correspondant à un état du patient permettant un enregistrement représentatif de son état clinique : rythme cardiaque suffisamment élevé, absence d'activité physique, absence de phase d'apnée ou d'hypopnée, absence d'arythmie, etc. cette liste n'étant pas limitative. Si l'un de ces critères n'est pas vérifié, la suite de l'analyse est inhibée, dans le cas contraire elle est poursuivie ;
- une période de délivrance de la stimulation contrôlée (ANS) ; et
- une période de récupération (RÉCUP), période pendant laquelle la stimulation ANS est arrêtée.

Le rythme cardiaque (intervalles RR) est mesuré pendant les périodes de ligne de base (BASE), de délivrance de la stimulation contrôlée (ANS) et de récupération (RÉCUP). En particulier, les informations recueillies au cours de la période de récupération, juste après la production de la salve ANS, sont significatives pour évaluer l'équilibre sympathovagal du patient, une fois qu'elles auront fait l'objet d'un traitement que l'on va maintenant décrire.

Les signaux mesurés pendant les différentes périodes peuvent être traités de façon intégrée ou indépendante.

On va décrire ci-après un *premier exemple d'un tel traitement,* permettant d'obtenir un indice SVB à partir d'une analyse de la seule période de récupération.

Le rythme cardiaque (intervalles RR) est mesuré à chaque période de récupération RÉCUP1, RÉCUP2... au cours de huit cycles cardiaques, donnant ainsi à chaque cycle huit échantillons qui sont mémorisés sous forme d'un vecteur {RR11, RR12 ... RR18} de huit valeurs ordonnées pour les mesures effectuées au cours de la phase de récupération RÉCUP1 de la première séquence SEQ1, et ainsi de suite pour les séquences suivantes SEQ2, SEQ3...

Une fois achevée la série SEQ1, SEQ2 ..., tous les échantillons de même rang qui ont été mémorisés sont moyennés, donnant ainsi un résultat de huit valeurs {RRm1, RRm2 ... RRm8} décrivant, sous forme moyennée, le profil de la courbe de variation de l'intervalle RR pendant la phase de récupération. La Figure 6 illustre ces différentes valeurs.

Ces données peuvent faire l'objet de diverses analyses permettant d'en extraire un indice SVB représentatif de l'équilibre sympathovagal du patient.

Dans l'exemple illustré Figure 7, les valeurs des réponses moyennées ont été représentées en fonction du rang i du cycle, donnant une caractéristique référencée 30. Pour quantifier la réponse autonomique, le dispositif recherche un ajustement de cette caractéristique 30 par une courbe, par exemple un modèle exponentiel 32, ou bien un modèle polynomial, etc. Les paramètres de cette courbe 32 sont alors extraits et combinés pour calculer un indice SVB représentatif. Si le modèle est par exemple une courbe d'équation RR = 1005,4 + 65,5786*exp(*tl*4,6869), on retiendra pour calculer l'indice SVB les trois valeurs {1005,4 ; 65,5786 ; 4,6869}. Dans un *second exemple de traitement,* le traitement est opéré à partir d'indicateurs issus d'une analyse portant à la fois sur la période de délivrance de la stimulation contrôlée (ANS) et sur la période de récupération (RÉCUP).

Plus précisément, ces deux périodes peuvent être décrites, comme indiqué plus haut, par une succession de quatre phases n° 1 à 4, avec consécutivement : diminution rapide du cycle cardiaque (intervalle RR) ou augmentation d'un autre paramètre notamment dérivé de la mesure de l'accélération endocardiaque (phase n° 1), pseudo-plateau (phase n° 2), augmentation rapide du cycle cardiaque à la fin de la période ANS (phase n° 3), et récupération proprement dite (phase n° 4).

Un traitement possible consiste à extraire de la série de valeurs observées les indicateurs suivants :
- pente et amplitude crête-à-crête de la série de valeurs pendant les phases n° 1 et n° 3, par :
   - détection et enregistrement d'une valeur de référence *Ref_Value* avant le début de la stimulation (à partir d'une mesure isolée ou à partir d'une version localement filtrée de la série) ;
   - détection de la valeur maximale *Peak_Value* pendant la période de stimulation ;
   - calcul de la valeur absolue de (*Peak_Value* - *Ref_Value*) ;
   - ajustement d'un modèle linéaire entre *Peak_Value* et *Ref_Value* et calcul des coefficients correspondants ;
- mesure de la durée de la récupération lors des phases n° 3 et 4, sous forme d'un seuil relatif par rapport à *Ref_Value* ou *Peak_Value ;*
- évaluation de la différence d'amplitude par rapport à *Ref_Value* ou *Peak_Value* pour N battements suivant la fin de la stimulation ;
- recherche d'un ajustement par une courbe qui peut être un modèle polynomial, exponentiel, etc. (comme exposé plus haut à propos de la Figure 7), l'erreur d'ajustement étant alors utilisé comme indicateur additionnel.

Ces divers indicateurs sont ensuite combinés entre eux pour en extraire un indice SVB courant, représentatif de l'équilibre sympathovagal du patient à un moment donné.

## Revendications

1. Un ensemble pour l'évaluation de l'équilibre sympathovagal, SVB, d'un patient, cet ensemble comprenant un dispositif médical actif comportant :
- un générateur (12) apte à produire des salves d'impulsions contrôlées de stimulation kinesthésique ;
- au moins un effecteur kinesthésique (14) configuré pour être appliqué sur un site cutané externe du patient, et comprenant un transducteur électromécanique vibrant configuré pour recevoir les impulsions produites par le générateur et délivrer une énergie de stimulation kinesthésique donnée à une fréquence de stimulation de 250 Hz ;
l'effecteur kinesthésique (14) étant configuré pour produire sur le site cutané externe du patient une stimulation mécanique vibratile qui est détectable par les récepteurs sensoriels ou mécanorécepteurs cutanés du patient ;et
- des moyens (16) de recueil d'au moins un signal physiologique représentatif de l'activité cardiaque, l'ensemble étant configuré tel que le au moins un signal physiologique recueilli par les moyens (16) de recueil permet de piloter le générateur (12) afin que celui-ci puisse délivrer à l'effecteur kinesthésique (14) des salves d'impulsions ANS ;
- des moyens d'activation contrôlée du générateur à intervalles programmés, pour la production pendant une période de test prédéterminée de salves de stimulation kinesthésique (28) ;
- des moyens de mesure, pour chaque salve de stimulation kinesthésique, d'au moins un paramètre témoin de l'activité autonomique courante du patient, dérivé du au moins un signal physiologique recueilli ;
- des moyens de calcul de la variation du au moins un paramètre témoin résultant de la stimulation kinesthésique, cette variation étant calculée à partir de valeurs dudit au moins un paramètre témoin comprenant au moins deux valeurs parmi :
i) une valeur de base mesurée pendant une période (BASE) en régime stationnaire antérieure à la production de la salve d'impulsions de stimulation kinesthésique ;
ii) une valeur courante mesurée pendant une période (ANS) de production de la salve d'impulsions de stimulation kinesthésique ; et
iii) une valeur courante mesurée pendant une période (RÉCUP) de récupération postérieure à la production de la salve d'impulsions de stimulation kinesthésique ; et
- des moyens évaluateurs, aptes à déterminer un indice SVB en fonction des variations du au moins un paramètre témoin résultant de la stimulation kinesthésique, calculées pendant ladite période de test prédéterminée.

2. L'ensemble de la revendication 1, dans lequel :
- pendant la période de test, le générateur est configuré pour produire une pluralité N de salves d'impulsions de stimulation kinesthésique ;
- pendant une période incluant ladite période de récupération, les moyens de mesure sont aptes à mesurer la valeur courante du au moins un paramètre témoin en synchronisme avec le rythme cardiaque (26) du patient pendant une pluralité de cycles cardiaques successifs, donnant pour chaque salve n échantillons distincts du au moins un paramètre témoin de rang i respectif, avec i = 1...n ; et
- les moyens évaluateurs sont aptes à calculer une moyenne des N échantillons de même rang i du au moins un paramètre témoin pour une même période de test, donnant ainsi n valeurs moyennées du paramètre témoin, l'ensemble de ces n valeurs moyennées étant l'indice SVB de la période de test.

3. L'ensemble de la revendication 1, comprenant en outre :
- des moyens aptes à inhiber les moyens d'activation contrôlée du générateur en cas de détection par le dispositif de la survenue d'au moins un événement parmi : fréquence cardiaque inférieure à un seuil prédéterminé, présence d'une activité physique du patient, délivrance d'une stimulation cardiaque, présence d'un épisode d'apnée ou d'hypopnée, présence d'un épisode d'arythmie.

4. L'ensemble de la revendication 1, dans lequel le paramètre témoin de l'activité autonomique courante du patient est au moins l'un d'entre : l'intervalle RR comme paramètre chronotropique ; l'intervalle PR comme paramètre dromotropique ; et un paramètre d'accélération endocardiaque EA comme paramètre inotropique.

## Patentansprüche

1. Kit zur Bewertung des sympathovagalen Gleichgewichts, SVB, eines Patienten, wobei das Kit eine aktive medizinische Vorrichtung folgendes umfasst:
- einen Generator (12), der in der Lage ist, Ausbrüche von gesteuerten kinästhetischen Stimulationsimpulsen zu erzeugen;
- mindestens einen kinästhetischen Effektor (14), der konfiguriert ist, um auf eine externe Hautstelle des Patienten aufgebracht zu werden, und der einen schwingenden elektromechanischen Wandler umfasst, der konfiguriert ist, um die vom Generator erzeugten Impulse zu empfangen und eine gegebene kinästhetische Stimulationsenergie bei einer Stimulationsfrequenz von 250 Hz abzugeben;
wobei der kinästhetische Effektor (14) konfiguriert ist, um an der äußeren Hautstelle des Patienten eine vibrierende mechanische Stimulation zu erzeugen, die durch die sensorischen oder mechanorezeptorischen Hautrezeptoren des Patienten nachweisbar ist; und
- Mittel (16) zum Sammeln mindestens eines physiologischen Signals, das für die Herzaktivität repräsentativ ist, wobei das Kit so konfiguriert ist, dass das mindestens eine physiologische Signal, das von den Sammelmitteln (16) gesammelt wird, es ermöglicht, den Generator (12) so zu steuern, dass er Ausbrüche von ANS-Impulsen an den kinästhetischen Effektor (14) abgeben kann;
- Mittel zur gesteuerten Aktivierung des Generators in programmierten Intervallen zur Erzeugung von kinästhetischen Stimulationsausbrüchen (28) während einer vorbestimmten Testzeit;
- Mittel zum Messen mindestens eines Steuerungsparameters der aktuellen autonomen Aktivität des Patienten, der sich aus dem mindestens einen gesammelten physiologischen Signal ableitet, für jeden Impuls der kinästhetischen Stimulation;
- Mittel zum Berechnen der Variation des mindestens einen Steuerungsparameters, der sich aus der kinästhetischen Stimulation ergibt, wobei diese Variation aus den Werten des mindestens einen Steuerungsparameters berechnet wird, der mindestens zwei Werte umfasst unter:
(i) einem Basiswert, der über einen Zeitraum (BASE) im stationären Zustand vor der Erzeugung des Ausbruchs von kinästhetischen Stimulationsimpulsen gemessen wurde;
(ii) einem Stromwert, der während eines Zeitraums (ANS) der Erzeugung des Ausbruchs von kinästhetischen Stimulationsimpulsen gemessen wurde; und
(iii) einem Stromwert, der während einer Erholungsperiode (RECOVERY) nach der Erzeugung des Ausbruchs von kinästhetischen Stimulationsimpulsen gemessen wird; und
- Auswertemittel, die in der Lage sind, einen SVB-Index als Funktion der Variationen des mindestens einen Steuerparameters zu bestimmen, der sich aus der kinästhetischen Stimulation ergibt, die während der vorbestimmten Testzeit berechnet werden.

2. Das Kit von Anspruch 1, worin:
- während der Testphase ist der Generator konfiguriert, um eine Vielzahl von N Ausbrüchen von kinästhetischen Stimulationsimpulsen zu erzeugen;
- während eines Zeitraums, der die Erholungsperiode einschließt, die Messmittel in der Lage sind, den aktuellen Wert des mindestens einen Steuerparameters synchron mit der Herzfrequenz (26) des Patienten über eine Vielzahl von aufeinanderfolgenden Herzzyklen zu messen, wobei für jeden Ausbruch n separate Proben des mindestens einen Steuerparameters des jeweiligen Ranges i mit i = 1...n gegeben werden; und
- die Auswertemittel in der Lage sind, einen Mittelwert aus den N Proben des gleichen Ranges i des mindestens einen Steuerparameters für den gleichen Prüfzeitraum zu berechnen, wodurch n Durchschnittswerte des Steuerparameters erhalten werden, wobei alle diese n Durchschnittswerte der SVB-Index für den Prüfzeitraum sind.

3. Das Kit nach Anspruch 1, ferner umfassend:
- Mittel, die in der Lage sind, die Mittel zur kontrollierten Aktivierung des Generators zu hemmen, falls die Vorrichtung das Auftreten mindestens eines der folgenden Ereignisse erkennt: Herzfrequenz unterhalb eines vorbestimmten Schwellenwerts, Vorhandensein einer körperlichen Aktivität des Patienten, Abgabe einer Herzstimulation, Vorhandensein einer Apnoe oder Hypopnoe-Episode, Vorhandensein einer Arrhythmie-Episode.

4. Das Kit nach Anspruch 1, worin der Steuerungsparameter der aktuellen autonomen Aktivität des Patienten mindestens einer von folgenden ist: das RR-Intervall als chronotroper Parameter; das PR-Intervall als dromotroper Parameter; und ein endokardialer Beschleunigungsparameter EA als inotroper Parameter.

## Claims

1. An assembly for evaluating the sympathovagal balance, SVB, of a patient, which assembly includes an active medical device having:
- a generator (12) capable of producing bursts of controlled kinesthetic stimulation pulses;
- at least one kinesthetic effector (14) configured to be applied to an external skin site of the patient, and comprising a vibrating electromechanical transducer configured to receive the pulses produced by the generator and deliver a given kinesthetic stimulation energy at a stimulation frequency of 250 Hz;
the kinesthetic effector (14) being configured to produce at the patient's external skin site a vibrating mechanical stimulation that is detectable by the patient's sensory or mechanoreceptor skin receptors; and
- means (16) for collecting at least one physiological signal representative of cardiac activity, the assembly being configured such that the at least one physiological signal collected by the collecting means (16) makes it possible to control the generator (12) so that it can deliver bursts of ANS pulses to the kinesthetic effector (14);
- means for controlled activation of the generator at programmed intervals, for the production during a predetermined test period of kinesthetic stimulation bursts (28);
- means for measuring, for each burst of kinesthetic stimulation, at least one control parameter of the patient's current autonomic activity, derived from the at least one physiological signal collected;
- means for calculating the variation of said at least one control parameter resulting from kinesthetic stimulation, this variation being calculated from values of the at least one control parameter comprising at least two values among:
i) a base value measured over a period (BASE) at steady state prior to the production of the burst of kinesthetic stimulation pulses;
ii) a current value measured during a period (ANS) of production of the burst of kinesthetic stimulation pulses; and
iii) a current value measured during a recovery period (RECOVERY) after the production of the burst of kinesthetic stimulation pulses; and
- evaluating means, capable of determining an SVB index as a function of the variations of the at least one control parameter resulting from kinesthetic stimulation, calculated during said predetermined test period.

2. The assembly of claim 1, wherein:
- during the test period, the generator is configured to produce a plurality N of bursts of kinesthetic stimulation pulses;
- during a period including said recovery period, the measuring means are capable of measuring the current value of the at least one control parameter in synchronism with the patient's heart rate (26) over a plurality of successive heart cycles, giving for each burst n separate samples of the at least one control parameter of respective rank i, with i = 1...n; and
- the evaluating means are able to calculate an average of the N samples of the same rank i of the at least one control parameter for the same test period, thus giving n average values of the control parameter, all these n average values being the SVB index for the test period.

3. The assembly of claim 1, further comprising:
- means capable of inhibiting the means of controlled activation of the generator in the event that the device detects the occurrence of at least one of the following events: heart rate below a predetermined threshold, presence of physical activity of the patient, delivery of cardiac stimulation, presence of an apnea or hypopnea episode, presence of an arrhythmia episode.

4. The assembly of claim 1, wherein the control parameter of the patient's current autonomic activity is at least one of: the RR interval as a chronotropic parameter; the PR interval as a dromotropic parameter; and an endocardial acceleration parameter EA as an inotropic parameter.
